# EUROPEAN PATENT APPLICATION

(11) **EP 1 890 145 A1**
(43) Date of publication of application: **20.02.2008**
(21) Application number: 06732552.2
(22) Date of filing: 12.05.2006
(51) Int. Cl.: G01N 33/53, G01N 21/78

(54) **INTERACTION DETECTION UNIT HAVING ELECTRODE OF THE SAME POTENTIAL, SENSOR CHIP USING THE DETECTION UNIT, AND INTERACTION DETECTION DEVICE**

(30) Priority: 03.06.2005 JP 2005164591
(71) Applicant: Sony Corporation, Minato-ku Tokyo 108-0075 (JP)
(72) Inventor: SEGAWA, Yuji, Shinagawa-ku, Tokyo 141-0001 (JP); KATSUMOTO, Yoichi, Shinagawa-ku, Tokyo 141-0001 (JP)
(74) Representative: Davies, Simon Robert
(86) International application number: PCT/JP2006/309571
(87) International publication number: WO 2006/129464

(57) **Abstract**

There are provided an interaction detecting portion including an interaction region in the whole of which an electrodynamic effect is obtained, and which has a simpler structure, and the like. There is provided an interaction detecting portion 1 including at least: a reaction region 2 which provides a field for an interaction between materials, and an electrode E₁ (or a group of electrodes), having the same potential, which is provided so as to face the reaction region 2. In addition, there are provided a sensor chip, such as a DNA chip or a protein chip, including the interaction detecting portion 1, and an interaction detector using the detection portion 1.

## Description

### [Technical Field]

The present invention relates to a technique for detecting an interaction between materials. More specifically, the invention relates to a technique for detecting an interaction between materials by utilizing an electrodynamic action.

### [Background Art]

In recent years, an integrated substrate for bioassay so-called a DNA chip or a DNA microarray (hereinafter generally referred to as "a DNA chip" in this application) in which predetermined DNAs are finely arranged by utilizing a microarray technique has been utilized for an analysis of mutation of a gene, an analysis of SNPs (single nucleotide polymorphisms), an analysis of a frequency of genomic expression, and the like. Also, the DNA chip begins to be generally used in the fields of a drug discovery, a clinical diagnosis, a pharmacogenomics, a research of evolution, a forensic medicine, and the others.

The feature of this "DNA chip" is that an exhaustive analysis of a hybridization becomes possible because the various and multiple DNA oligo chains or nucleotide chains such as a complementary DNA (cDNA) are integrated on a glass substrate or a silicon substrate. In addition thereto, a sensor chip (for example, a protein chip) for detecting an interaction between biologic molecules other than nucleic acid molecules or a detector is variously developed.

Here, a technique utilizing an electrodynamic effect such as an electrophoresis or a dielectrophoresis in an assay system for causing an interaction between materials to progress, and detecting the interaction in a predetermined reaction region has recently began to be proposed. Hereinafter, this technique will be exemplified.

Firstly, Japanese Patent Laid-Open No. 2001-330608 discloses a technique that a nucleic acid probe template chain immobilized on a template substrate is used to synthesize a nucleic acid probe chain along the template chain, and the synthesized probe chain is immobilized on another array substrate by utilizing an electric field, thereby simply manufacturing a nucleic acid chain immobilized array at a low cost.

Japanese Patent Laid-Open No. 2001-242135 discloses a technique that a chip for detection is composed of a main body portion and a frame which are detachable from each other, a large number of pin electrodes are protruded in matrix inside the main body portion, oligonucleotide composed of different gene sequences is immobilized on the pin electrodes, a common electrode is disposed in a hollow of the frame so as not to contact any of the pin electrodes, a voltage is applied across the common electrode and each of the pin electrodes, thereby detecting a double-strained DNA obtained through a hybridization by detecting a current.

Japanese Patent Laid-Open No. 2004-135512 discloses a hybridization detecting portion structured such that a reaction region becoming a field for a hybridization between a nucleotide chain for detection and a target nucleotide chain having a base sequence complementary to the nucleotide chain for detection can be fixedly fastened to an end portion of a scanning electrode through an action of a dielectrophoresis while the nucleotide chain for detection is elongated by application of an electric field.

Each of the prior arts described above utilizes the electrode or a group of electrodes which is disposed so as to face the reaction region. Therefore, each of the prior arts described above involves a technical problem that it is difficult to exert the desired electrodynamic effect on the entire reaction region because the electric field (electric lines of force) is generated across the counter electrodes.

In addition, the reaction region in the sensor chip such as the DNA chip or the protein chip is generally limited. Hence, when the electrode or the group of electrodes showing the counter disposition relationship with the reaction region are provided, there are encountered technical problems: (1) The structure of the reaction region becomes more complicated, (2) the morphologic restriction to the reaction region occurs because of the formation of the counter electrode, (3) the number of processes for manufacturing the chip increases, (4) the distribution- of the electric supply line to the electrodes is also complicated, and so forth.

In the light of the foregoing, it is a principal object of the present invention to provide an interaction detecting portion which is capable of enabling an electrodynamic effect to be obtained in an entire reaction region, and which includes a reaction region having a simpler structure, and the like.

### [Disclosure of the Invention]

The present invention firstly provides an interaction detecting portion including at least a reaction region for providing a field for an interaction between materials, and an electrode or a group of electrodes, having the same potential, which is provided so as to face the reaction region, and a sensor chip including at least one interaction detecting portion concerned. An A.C. electric field, for example, is adopted as an electric field applied to the electrode or the group of electrodes, thereby obtaining a desired electrodynamic effect such as a dielectrophoresis.

In addition, adoption of a structure in which the electrode or the group of electrodes has an irregular shape in its surface makes it possible to form a nonuniform electric field in the irregular shape, especially, in the vicinity of a stepped portion. An action of the dielectrophoresis can be efficiently obtained in a region in which the nonuniform electric field is formed. In addition thereto, the surface of the electrode or the group of electrodes is covered with an insulating layer, thereby making it possible to prevent an electrochemical reaction from occurring due to an ion solution which is accumulated in the reaction region in some cases.

The interaction which is made to progress in the reaction region is not construed in a limiting sense. However, the hybridization between the nucleic acid chains with which it is expected that the action of the dielectrophoresis gives the efficiency and precision of the interaction the better effect can be given as an example of the interaction described above.

Next, the present invention provides an interaction detector including at least a reaction region for providing a field for an interaction between materials, an electrode or a group of electrodes, having the same potential, which is provided so as to face the reaction region, and means for applying an electric field to the electrode or the group of electrodes.

Means for applying an A.C. electric field across the electrode or the group of electrodes, and a grounding portion, for example, can be adopted as the electric field applying means constituting the interlayer detector. In addition, the electric field applying means can also apply a high frequency electric field to the electrode or the group of electrodes through an impedance matching circuit.

With the interaction detector, the electrodynamic action such as the dielectrophoresis is given to the material existing in the reaction region by application of the electric field.

Here, the main technical terms relating to the present invention will now be described.

"The interaction" generally means a chemical bond or dissociation containing a noncovalent bond, a covalent bond and a hydrogen bond between materials. For example, "the interaction" generally contains the hybridization between nucleic acid molecules, the interaction between protein materials, and the chemical bond or dissociation, between materials, such as an antigen-antibody complex reaction. It is noted that "the hybridization" means a complementary strand (duplex) forming reaction, between materials, having a complementary base sequence structure.

"The nucleic acid chain" means a polymer (nucleotide chain) of phosphate ester of nucleoside obtained by glycosidically bonding a purine or pyrimidine base and sugar to each other. Thus, "the nucleic acid chain" generally contains a DNA (an entire length or a fragment) obtained by polymerizing oligonucleotide containing a prove DNA, a polynucleotide, purinenucleotide, and pyrimidinenucleotide, a cDNA (c prove DNA) obtained through reverse transcription, an RNA, a polyamidenucleotide derivative (PNA), and the like.

"The reaction region" is a region in which the field for the interaction such as the hybridization can be provided. A reaction field, having a well shape, in which a liquid phase material or a gel can be accumulated is given as an example of "the reaction region".

"The dielectrophoresis" is a phenomenon that molecules are driven to a stronger electric field in a field having a nonuniform electric field. Even when the A.C. voltage is applied to the molecules, the driving effect can be obtained similarly to the case of application of the D.C. voltage because the polarity of the polarization is also inversed along with inversion of the polarity of the applied voltage (refer to a literature of "Micro Machine and Materials (published by CMC Publishing Co., Ltd.)" complied under the super vision of Teru Hayashi, Manipulation of Cells and DNAs (Fifth Chapter), pp.37 to 46). In particular, the force acts on the dipoles of the molecules in proportion to a gradient of a square of a time average electric field within the high frequency A.C. electric field, so that the molecules migrate.

For example, it is known that when receiving the action of the electric field exerted thereon in the liquid phase, the nucleic acid molecules elongate or move. With regard to the principles thereof, it is thought that phosphoric acid ions (negative electric charges) constituting a skeleton, and hydrogen atoms (positive electric charges) into which the water lying in the vicinity of the phosphoric acid ions are ionized form an ionic cloud together with each other. Also, the polarization vectors (dipoles) generated from the negative electric charges and the positive electric charges are oriented to one direction as a whole by application of the high voltage having a high frequency to elongate. In addition thereto, when the nonuniform electric field is applied to the nucleic acid molecules, the polarization vectors move toward the portion on which the electric lines of force concentrate (refer to a literature of Seiichi Suzuki, Takeshi Yamanashi, Shinichi Tazawa, Osamu Kurosawa and Masao Washizu: "Quantitative Analysis on Electrostatic Orientation of DNA in Stationary AC Electric Field Using Fluorescence Anisotropy", IEEE Transaction on Industrial Application, Vol. 34, No. 1, pp. 75 to 83 (1998)).

"The sensor chip" means a substrate for causing an interaction between materials to progress, and detecting the interaction between the materials in a predetermined reaction region on the substrate. Thus, the sensor chip generally includes such substrates irrespective of the kind of material, and the principles for detecting the interaction is not subject matter in the sensor chip. The sensor chip at least includes the DNA chip (DNA microarray) in which the nucleic acid chains such as the DNA probe is immobilized on the substrate to become a state of being finely arranged, the protein chip which is suitable for detecting the interaction between the protein materials, and the antigen-antibody complex reaction or the like, and the like.

The present invention adopts the structure that the electrode (that is, the counter electrode) disposed so as to face the reaction region is not provided, but the electrode or the group of electrodes having the same potential is provided. As a result, the applied electric field (electric lines of force) can be made to diverge in all the directions in the vicinity of the electrode. That is to say, since the electric field (electric lines of force) can be made to diverge far and wide toward the entire reaction region, the electrical gradient, or the nonuniform electric field can be formed in the entire reaction region. As a result, the materials dispersed in the reaction region can be efficiently collected up in larger numbers to the electrode side through the electrodynamic migration action.

Since it is unnecessary to form the counter electrode in the reaction region, the number of morphologic restrictions to the reaction region is less, and thus the structure of the detection portion can be simplified. For example, in the electric field applying means, with respect to the power feeding wiring itself, the grounding portion has to be previously connected to the system side, and the wiring for the detection portion has to be connected to only the electrode in the hybridization region. As a result, the structure of the detector can also be simplified.

### [Brief Description of Drawings]

[FIG. 1] FIG. 1 is a main portion cubic perspective view explaining a basic constitution of a first embodiment as an interaction detecting portion (hereinafter referred to as "a detection portion") according to the present invention.
[FIG. 2] FIG. 2 is a transverse cross sectional view of the detection portion.
[FIG. 3] FIG. 3 is a cross sectional view taken on line A - A of FIG. 2.
[FIG. 4] FIG. 4 is a circuit diagram simply showing an example of a circuit structure of an impedance matching circuit (8).
[FIG. 5] FIG. 5 is a block diagram showing a more detailed constitution of a preferred embodiment of electric field applying means.
[FIG. 6] FIG. 6 is a cross sectional view (a cross sectional view common to FIG. 2) schematically showing a situation in which an electric field is applied to a reaction region (2) by using electric lines of force.
[FIG. 7] FIG. 7 is a perspective view showing a second embodiment of the detection portion according to the present invention.
[FIG. 8] FIG. 8 is a graph showing a distribution of electric field strength in a vertical direction when an A.C. electric field obtained from ± 20 V of 1 MHz is applied across an electrode E₁ and the ground.
[FIG. 9] FIG. 9 is a graph showing a distribution of electric field strength in an oblique direction when the A.C. electric field of FIG. 8 is applied.
[FIG. 10] FIG. 10 is a graph showing a distribution of electric field strength in a horizontal direction when the A.C. electric field of FIG. 8 is applied.
[FIG. 11] FIG. 11 is a graph showing a distribution of a percentage change of electric field strength (a gradient of a square of electric field strength) in a vertical direction.
[FIG. 12] FIG. 12 is a graph showing a distribution of a percentage change of electric field strength (a gradient of a square of electric field strength) in an oblique direction.
[FIG. 13] FIG. 13 is a graph showing a distribution of a percentage change of electric field strength (a gradient of a square of electric field strength) in a horizontal direction.
[FIG. 14] FIG. 14 is a drawing substitution data table showing electric field strength data (right-hand side) and numerical data (left-hand side) on differential calculus of a square of electric field strength with respect to distances (unit: µm) in a vertical direction, in an oblique direction, and in a horizontal direction, respectively.
[FIG. 15] FIG. 15 is a view showing concepts of the vertical direction (Z), the horizontal direction (H), and the oblique direction (R) with respect to the distributions of the electric field strength in a periphery of an electrode.

### [Best Mode for Carrying out the Invention]

Hereinafter, preferred embodiments for carrying out the present invention will be described with reference to the accompanying drawings. It is noted that each of embodiments shown in the accompanying drawings merely shows an example of a typical embodiment of a product or a method relating to the present invention, and thus the scope of the present invention is not intended to be construed in a limiting sense.

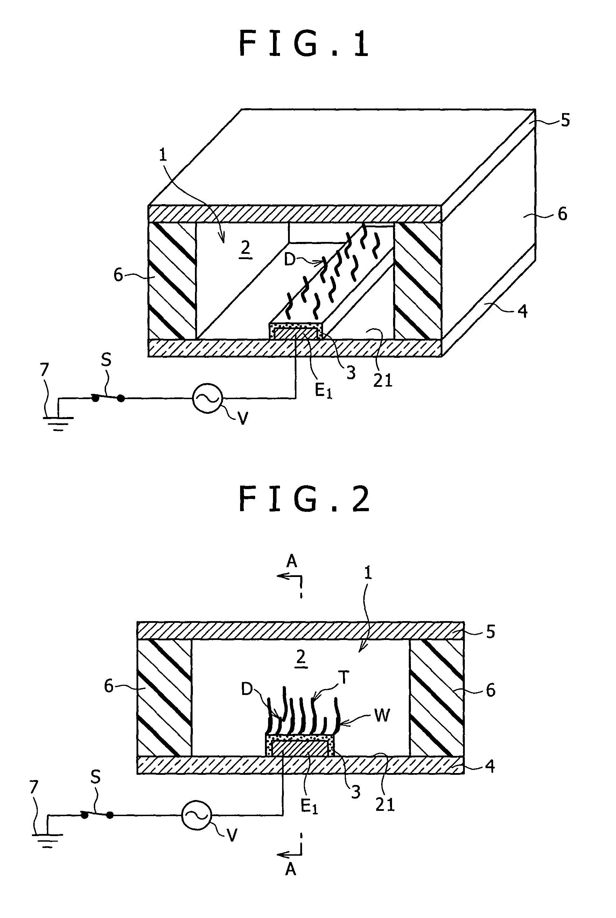

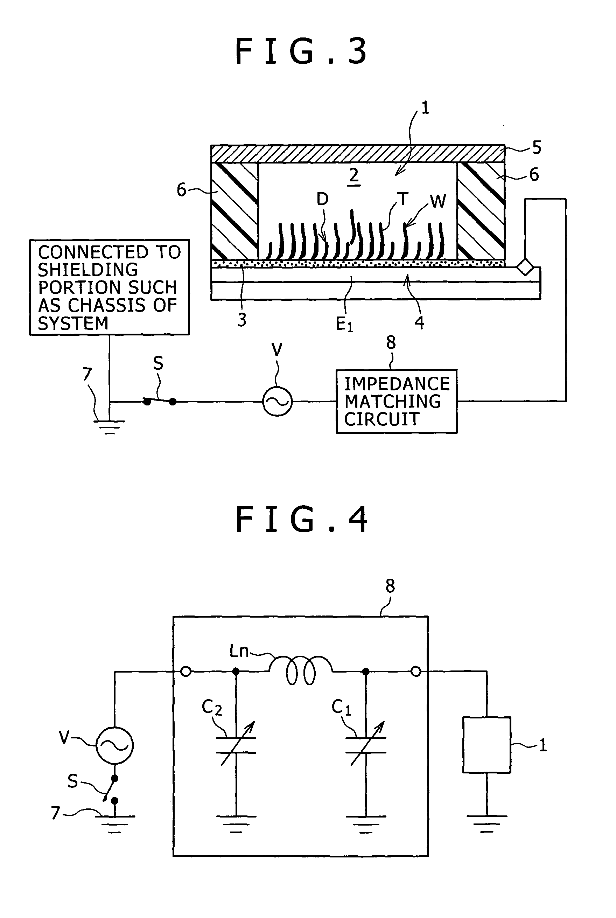

FIG. 1 is a main portion cubic perspective view explaining a basic constitution of a first embodiment as an interaction detecting portion (hereinafter referred to as "a detection portion") according to the present invention, FIG. 2 is a transverse cross sectional view of the detection portion, and FIG. 3 is a cross sectional view taken on line A - A of FIG. 2.

Firstly, FIGS. 1 to 3 show the preferred first embodiment of the detection portion 1 according to the present invention. As shown in these figures, a reaction region 2 having a well shape (inverse recess shape) in which a medium such as a solution or a gel can be accumulated or held is provided in the detection portion 1.

The reaction region 2 functions as a region or a space for providing a field for an interaction, such as a hybridization, between materials. An A.C. electric field, for example, is applied to the medium accumulated or held in the reaction region 2 through an electrode E₁ which is formed so as to face the reaction region 2.

Here, the electrode E₁ is made of either a metal such as aluminum or gold, or a transparent conductor such as ITO (indium-tin-oxide). In an example of this embodiment, the electrode E₁ is disposed at a central position of a bottom surface-21 of the reaction region 2. It is noted that the place where the electrode E₁ is formed is not limited to the bottom surface 21. For example, the electrode E₁ may also be formed either in an upper side substrate 5 or in a position of a spacer 6 which will be described later so as to face the reaction region 2.

As shown in FIGS. 1 to 3, the electrode E₁ is desirably covered with an insulating layer 3 made of a material which is selected from among SiO₂, SiC, SiN, SiOC, SiOF, TiO₂, and the like. The reason for this is because an electrochemical reaction is prevented from occurring due to an ion solution which is accumulated in the reaction region 2 in some cases.

A surface of the electrode E₁ can be operated as a detection surface on which a material D to be detected such as a DNA probe is immobilized. More specifically, surface processing for allowing the end of the material D to be detected such as the probe DNA to be immobilized is previously executed for the electrode E₁.

The material D to be detected can be immobilized on the surface of the electrode E₁ based on a coupling reaction or the like occurring between the surface of the electrode E₁ and the end of the probe DNA (an example of the material D to be detected). For example, in the case of the electrode surface for which the surface processing is executed by using streptavidin, it is suitable for immobilizing the end of the biotinylated material D to be detected. Or, in the case of the electrode surface for which the surface processing is executed by using a thiol (SH) group, it is suitable for immobilizing the material D to be detected, such as the probe DNA, having the end modified with the thiol group by using a disulfide bond (-S-S-bond).

It is noted that reference symbols D and T shown in each of FIG. 1 and the like schematically designate the material to be- detected, typified by the DNA probe, having the end immobilized on the surface of the electrode E₁, and a target material showing a specific interaction with the material D to be detected, respectively. In addition, reference symbol W shown in FIGS. 2 and 3 designates a complex (for example, a double-stranded nucleic acid) which is formed based on a specific interaction (for example, a hybridization) between the material D to be detected and the target material T.

Reference numerals 4 and 5 shown in each of FIGS. 1 to 3 designate substrates, respectively. The substrate 4, for example, is an optically-transparent substrate with which recorded information (interaction information in the present invention) within the reaction region can be optically read out, and for example, is made of a quartz glass or silicon, or a synthetic resin such as polycarbonate, polystyrene or polyolefin. On the other hand, the substrate designated with reference numeral 5 functions as a cover which closes the reaction region 2, and may be made of the same base material as that of the substrate 4, or may be made of a material having a light reflecting function in accordance with the intended use.

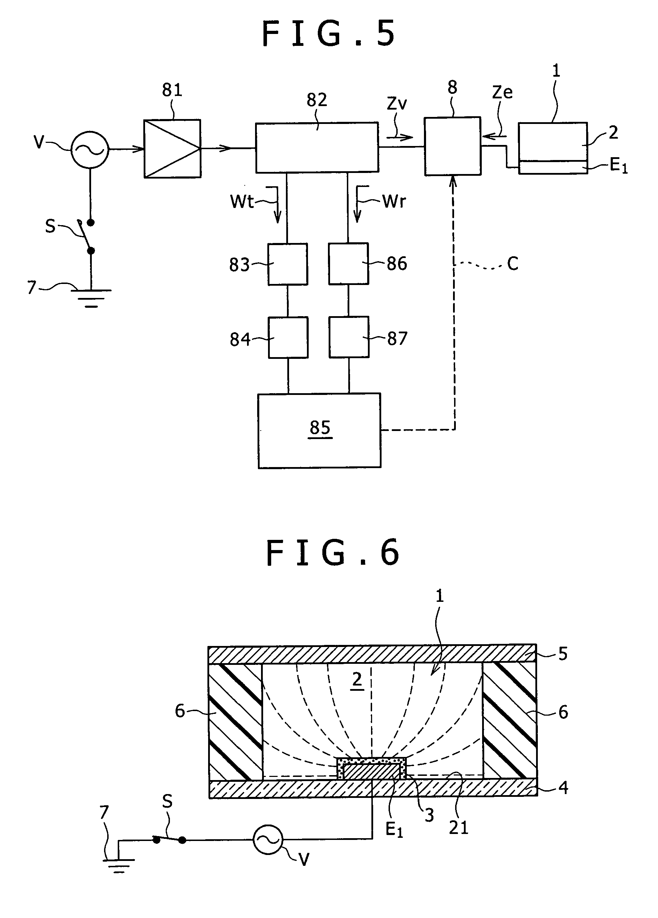

Reference numeral 6 shown in each of FIG. 6 and the like designates a spacer member made of an insulating material such as SiO₂ or a synthetic resin. It is noted that this spacer member 6 may be formed either separately from the substrate 4 or 5, or integrally with the substrate 4 or 5. When the spacer member 6 is formed integrally with the substrate 4 or 5, the reaction region 2 which plays a role for presenting a field for the interaction such as the hybridization can be formed by utilizing the known optical disc mastering technique.

Subsequently, a structure is adopted in which a voltage can be applied from a power source V such as an A.C. power source across the electrode E₁ and a grounding portion 7 provided outside the reaction region 2 in accordance with an operation for turning ON/OFF a switch S (electric field applying means).

With such a structure of the electric field applying means, since it is unnecessary to form a counter electrode corresponding to the electrode E₁ within the limited reaction region 2, the structure of the reaction region 2 can be simplified. Moreover, when the electric field is applied thereacross, with respect to the wiring itself, the grounding portion has to be previously connected to the system side, and the wiring to the detection portion has to be connected to only the electrode E₁ within the reaction region 2. As a result, the structure of the detector (for detecting the hybridization and a signal) can also be simplified.

When the switch S is turned ON, the electric field concentrates on the vicinity of the electrode E₁, so that the nonuniform electric field can be formed. With regard to the electrode structure suitable for generating the nonuniform electric field, while not especially illustrated, it is expected that the surface of the electrode E₁, for example, is subjected to rough surfacing processing so as to obtain an irregular shape, or is patterned so as to obtain an island-like shape, thereby causing the electric field to be easy to concentrate on a convex portion (angled portion) of the surface of the electrode E₁ concerned.

With such a structure, the electric field can be especially caused to concentrate on a corner portion of the convex portion, a bend portion of a recess portion, or the like. As a result, the action and effect of the dielectrophoresis can be more reliably utilized in the region in the vicinity of the electrode E₁. It is noted that although the method of subjecting the electrode surface to the rough surfacing processing can be carried out by, for example, utilizing the known sputter depositing technique, epitaxy depositing technique or etching technique, it is not especially limited.

Based on the action of the nonuniform electric field, the material which dispersively exists in the reaction region 2 described above at random can be drawn near the electrode E₁ side or can be elongated in a direction along the electric field due to the electrodynamic action such as the dielectrophoresis.

The strength, frequency, and an application period of time of the applied electric field are not especially limited. Thus, the proper electric field strength, frequency, and application period of time of the applied electric field are desirably selected in accordance with the kind, the molecular length and the like of the object to which the electric field is applied. Also, the waveform of the applied electric field is not also limited to the sine wave, and thus for example, may be a chopping wave or the like.

In the case where the A.C. electric field is actually applied to the electrode E₁, it is preferable that as shown in FIG. 3, an impedance matching circuit 8 is provided between the electrode E₁ and the power source V to obtain the impedance matching between them, so that the power can be efficiently supplied to the electrode E₁.

FIG. 4 simply shows an example of a circuit structure of the impedance matching circuit 8. The impedance matching circuit 8 is provided between the grounded power source V and the grounded detection portion 1. Also, the impedance matching circuit 8 includes two variable capacitors C₁ and C₂ which are grounded, and an inductance neutralizing circuit (neutralization coil) Lₙ, as a neutralization element, which is interposed between the two variable capacitors C₁ and C₂.

When the electric field is applied, the two variable capacitors C₁ and C₂ play a role for adjusting a capacitance, and the inductance neutralizing circuit Lₙ plays a role for adjusting an inductance. With such a structure, there is performed the impedance matching between an internal impedance outputted from the power source (signal generating source) V, and a load impedance of the power source.

Moreover, a more detailed constitution of a preferred embodiment of the electric field applying means is shown in the form of a block diagram in FIG. 5.

A signal outputted from the grounded power source (that is, the signal generating source) V is amplified by an amplifier 81 connected to the grounded power source V and is taken out in the form of an output signal. Also, a transmission wave Wₜ about the resulting output signal is inputted and detected by a signal detector (sensor) 83 through a directional coupler designated with reference numeral 82. Also, an output power of the output signal thus detected is measured by a measuring instrument 84. A control portion 85 monitors this measured value.

In addition, a reflected wave Wᵣ from the electrode E₁ located at a termination is detected by the signal detector (sensor) 86 through the directional coupler 82, and a reflected power thereof is measured by a measuring instrument 87. The control portion 85 monitors this measured value.

The impedance matching circuit 8 adjusts a magnitude of the internal impedance Zᵥ of the power source, and a magnitude of the load impedance Zₑ of the counter electrode E₁ in accordance with a control signal C which the control portion 85 outputs based on the measured value described above (refer to FIG. 5). As a result, the impedance matching circuit 8 performs the matching between both the impedances Zᵥ and Zₑ so as to obtain a relationship of Zᵥ = Zₑ. More specifically, either a capacitance component or an inductance component within the impedance matching circuit 8 is changed by, for example, the motor driving so that the reflected power measured in the control portion becomes the smallest one. In such a manner, the impedance automatic adjustment is carried out.

By the impedance matching means described above, the loss of the power supplied from the power source V to the counter electrode E₁ is reduced, so that the supplied power becomes maximum by utilizing the technique for exerting the electrodynamic action on the material existing in the reaction region 2 of the detection portion 1, for example, the nucleic acid molecules.

In addition, it is possible to reliably carry out the uniformity (stabilization) of the applied power (supplied power) to the reaction region 2, and moreover the suppression of a disturbance in a waveform of a pulse signal which occurs when the high frequency electric field or the A.C. electric field, especially, the high frequency A.C. electric field is utilized, the erasing of the reflected wave Wᵣ from the electrode E₁ causing a problem about a phase delay of the applied power or the like, and the like.

Giving a description by showing the case where the nucleic acid chain as the target material T exists in the reaction region 2 as an example, the nucleic acid chain concerned receives the electrodynamic action of the dielectrophoresis to migrate toward the electrode E₁ having the stronger electric field strength. As a result, the target nucleic acid chains are collected on the surface of the electrode E₁ on which the nucleic acid chains such as the probe DNA functioning as the material D to be detected are previously immobilized, so that the hybridization efficiently progresses.

That is to say, the target nucleic acid chains are moved to the surface of the electrode E₁ for a short period of time through the dielectrophosresis described above to increase the concentration thereof, which results in that a period of time required for the hybridization with the nucleic acid chains immobilized on the surface of the electrode E₁ can be greatly shortened.

Moreover, when the single stranded nucleic acid chain elongates resulting from the action of the dielectrophoresis, the steric hindrance in the phase of the hybridization is reduced as compared with the case of being curled up into a random coil state. As a result, it is possible to enhance the efficiency of the hybridization.

It is noted that with respect to a signal about the hybridization, a quantity of light emitted from an intercalate which is specifically bonded to the double strand (double stranded nucleic acid) may be measured, a quantity of light emitted from a fluorescence dye which is previously bonded to the target DNA may be measured after the excessive DNAs are removed after completion of the hybridization. Or, a quantity of emitted light following the hybridization reaction may be measured by utilizing a molecular beacon.

Here, FIG. 6 is a cross sectional view (a cross sectional view common to FIG. 2) in which a situation of applying the electric field to the reaction region 2 is schematically shown by using the electric lines of force.

In the detection portion 1 according to the present invention, as shown in FIG. 6, the electric field is formed from the electrode E₁ having no counter electrode toward the entire reaction region 2. As a result, the desired electrodynamic action such as the dielectrophoresis can be exerted on the entire reaction region 2.

That is to say, since the electric field (electric lines of force) can be made to diverge extensively toward the entire reaction region, the electrical gradient or the nonuniform electric field can be formed throughout the reaction region 2. As a result, the material dispersed in the reaction region 2 can be efficiently collected up in larger numbers on the electrode E₁ side based on the electrodynamic migration action.

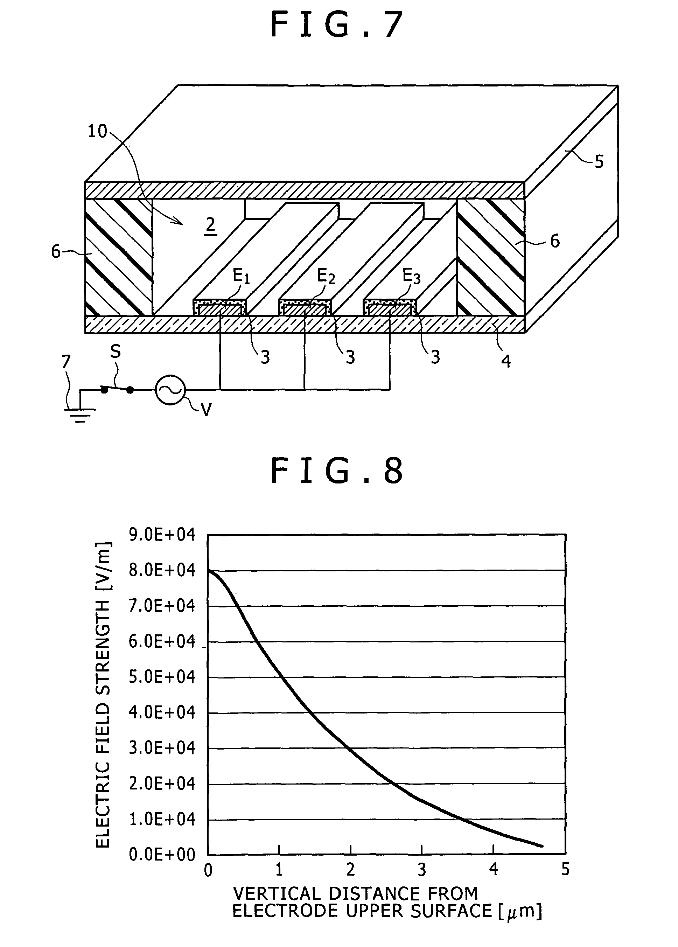

FIG. 7 is a view showing a second embodiment of a detection portion according to the present invention. The detection portion 10 as the second embodiment includes three electrodes E₁, E₂ and E₃ having the same potential. The number of electrodes having the same potential may be two, or three or more. Also, it is also free that a plurality of electrodes are disposed on the upper surface of the substrate 4, or the like in various patterns.

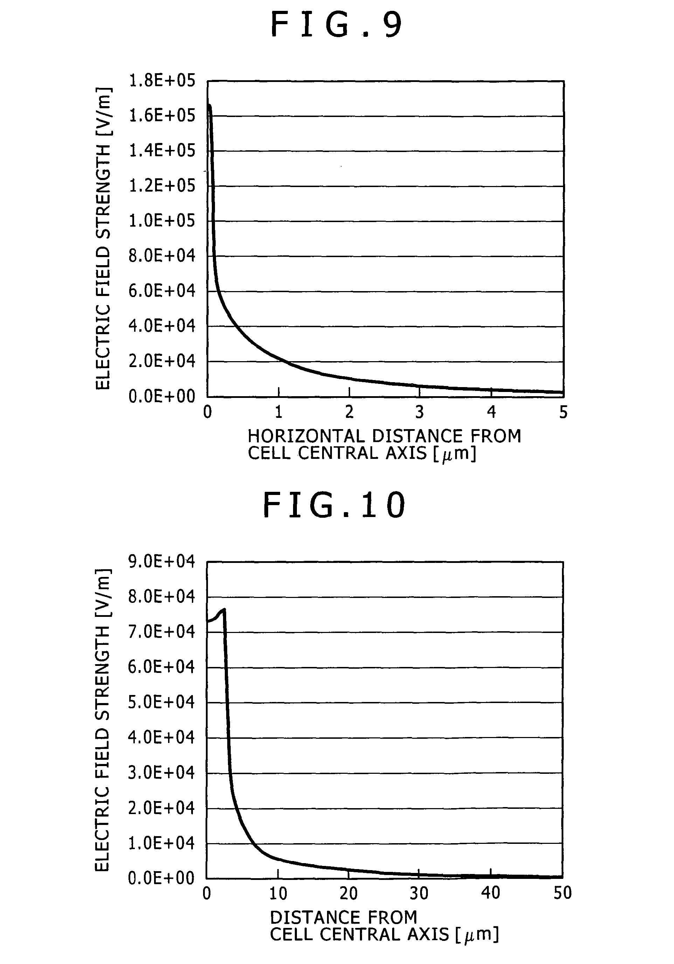

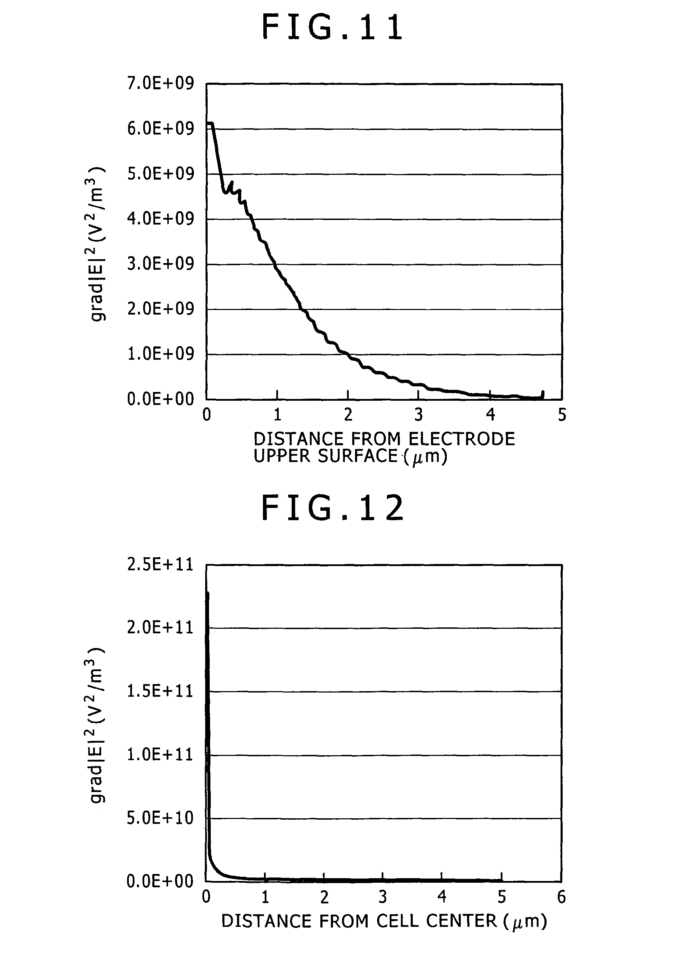

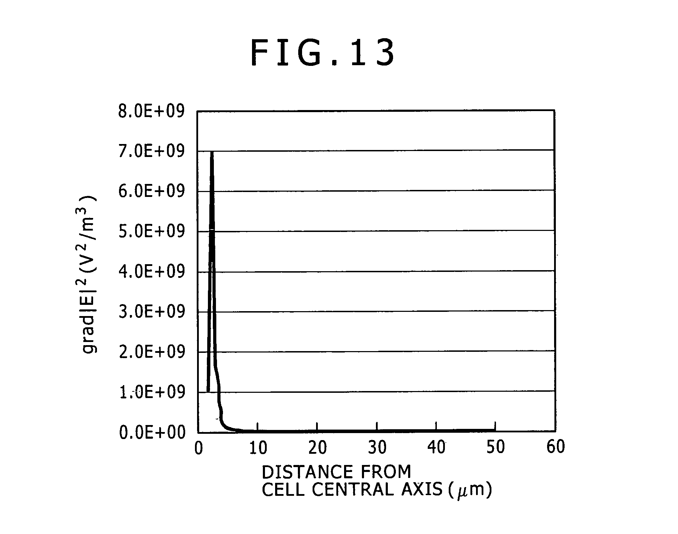

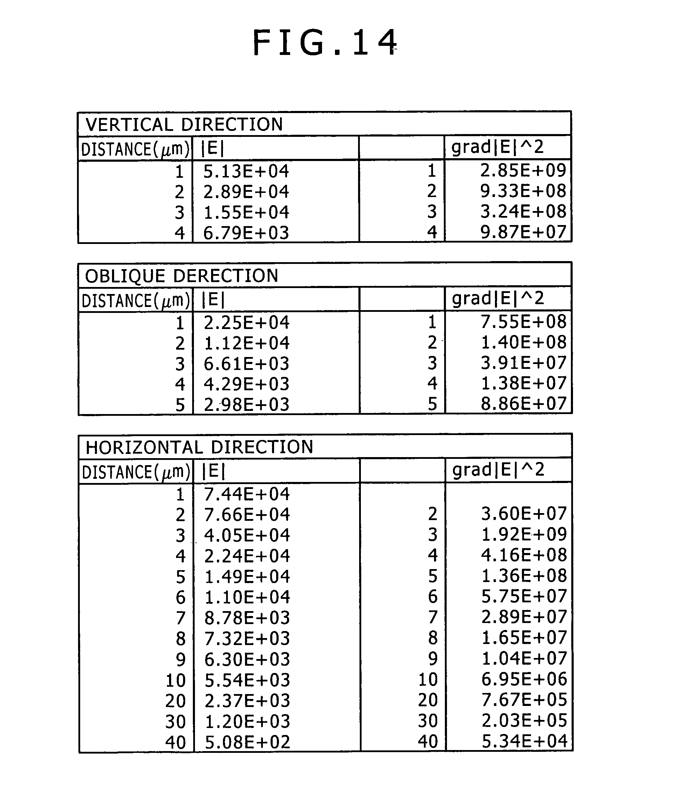

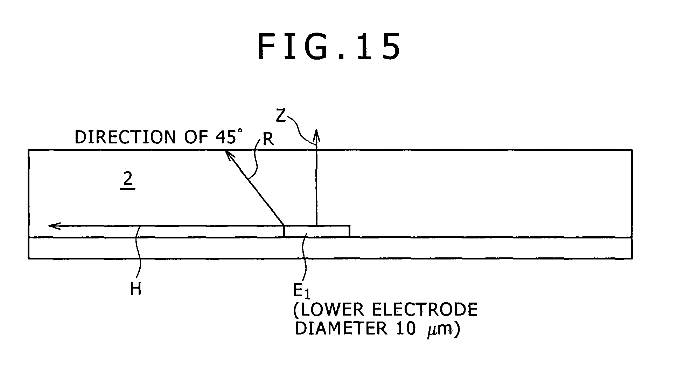

Here, FIGS. 8, 9 and 10 show the distributions of the electric field strength in a vertical direction, in an oblique direction, and in a horizontal direction, respectively, in the case where the A.C. electric field obtained from ± 20 V of 1 MHz is applied across the electrode E₁ and the ground on the assumption that the grounding portion is provided at infinity in the electrode E₁ having a diameter of 10 µm provided in the reaction region which is 100 µm in diameter and 5 µm in height. In addition, FIGS. 11, 12 and 13 show distributions of the percentage changes of the electric field strength (the gradients of squares of the electric field strength) in the vertical direction, in the oblique direction and in the horizontal direction, respectively. In addition, FIG. 14 is a drawing substitution data table showing electric field strength data (right-hand side) and numerical data (left-hand side) on differential calculus of a square of electric field strength with respect to distances (unit: µm) in the vertical direction, in the oblique direction, and in the horizontal direction, respectively. It is noted that FIG. 15 is a view showing concepts of the vertical direction (Z), the horizontal direction (H), and the oblique direction (R) with respect to the distributions of the electric field strength in the periphery of the electrode.

It is understood from the results represented in these drawings that the electric field is formed within the reaction region, the electric field strength is larger in the vicinity of the electrode (E₁), and thus the nonuniform electric field is generated. Note that, when the A.C. electric field is actually applied, as described above, it is preferable that the impedance matching is obtained, thereby allowing the power to be efficiently applied.

### [Industrial Applicability]

The present invention can be utilized as the technique for detecting efficiently and precisely the interaction between the materials for a short period of time by utilizing the electrodynamic action. Also, the present invention can be utilized as the technique for the sensor chip typified by the DNA chip or the protein chip, or as the apparatus for detecting the interaction.

## Claims

1. An interaction detecting portion, comprising:
a reaction region which provides a field for an interaction between materials; and
an electrode or a group of electrodes, having the same potential, which is provided so as to face said reaction region.

2. The interaction detecting portion according to claim 1, wherein an alternate current electric field is applied to said electrode or group of electrodes.

3. The interaction detecting portion according to claim 1, wherein a surface of said electrode or group of electrodes has an irregular shape.

4. The interaction detecting portion according to claim 1, wherein a surface of said electrode or group of electrodes is covered with an insulating layer.

5. The interaction detecting portion according to claim 1, wherein the interaction is a hybridization between nucleic acid chains.

6. A sensor chip comprising at least one interaction detecting portion according to claim 1.

7. An interaction detector, comprising:
a reaction region which provides a field for an interaction between materials;
an electrode or a group of electrodes, having the same potential, which is provided so as to face said reaction region; and
electric field applying means for said electrode or group of electrodes.

8. The interaction detector according to claim 7, wherein said electric field applying means is means for applying an A.C.- electric field across said electrode or group of electrodes and a grounding portion.

9. The interaction detector according to claim 7, wherein said electric field applying means applies a high frequency electric field through an impedance matching circuit.

10. The interaction detector according to claim 7, wherein an electrodynamic action is exerted on a material existing in said reaction region by application of an electric field.

11. The interaction detector according to claim 9, wherein the electrodynamic action is an electrophoresis and/or a dielectrophoresis.
